# EUROPEAN PATENT APPLICATION

(11) **EP 2 204 150 A1**
(43) Date of publication of application: **07.07.2010**
(21) Application number: 08173092.1
(22) Date of filing: 30.12.2008
(51) Int. Cl.: A61F 7/02, A61F 7/10

(54) **Long-acting cold structure**

(71) Applicant: Lu, Nan Chih, Pingzhen City (TW)
(72) Inventor: Lu, Nan Chih, Pingzhen City (TW)
(74) Representative: Jeannet, Olivier

(57) **Abstract**

The present invention relates to a long-acting cold pack (1) structure for cold compress therapy on human body. The cold pack (1) is composed of a cold maintaining layer (2), a fluid layer (3) and an air bag layer (4) which are stacked in sequence. The cold maintaining layer (2) is filled with gel-like cold maintaining material. The fluid layer (3) is adapted for circulating low temperature fluid. The air bag layer (4) is filled with air as desired. With the circulation of the low temperature fluid in the fluid layer (3), the present invention permits the cold maintaining material in the cold maintaining layer (2) to extend the time at a low temperature. The air bag layer (4) is filled with air to compress the cold maintaining layer (2) for depth cold compress therapy. The cold pack (1) of the present invention provides better cold compress effect than the prior art.

## Description

### BACKGROUND OF THE INVENTION

### 1. Field of the Invention

The present invention relates to a long-acting cold pack structure for better cold compress effect, and more particularly to one that keeps the pack for a long time at a low temperature and fits for various usage.

### 2. Description of the Prior Art

Cold compress therapy is verified to have a curative effect. The principle of the cold compress therapy is to lower the temperature of the surface of the affected part of a patient's body for vasoconstriction so as to reduce pain and swelling from a sports or activity injury to soft tissues. The therapy is especially useful for sprains, strains, pulled muscles and pulled ligaments.

A traditional cold compress therapy uses a bag filled with ice cubes or iced water and covered with a towel. This is not convenient in use and the temperature is not low enough. In addition, it is hard to keep the low temperature for a long time.

A conventional cold pack is filled with gel-like cold maintaining material which is composed of water, sodium, acid, CH₃OH, cellulose, and pigment. The cold pack must be frozen for several hours in advance and taken out from the refrigerator for usage as desired, keeping the cold pack for a certain time at a subzero temperature. The temperature of the cold pack is raised slowly. This cold pack is popularly used. However, the limitation of this cold pack is that the cold pack must be frozen in advance. Besides, the user must press the cold pack for depth cold compress therapy. This is inconvenient in use.

Another conventional cold pack includes an inlet and an outlet coupled with a pipe for the circulation of low temperature fluid. The advantage of this cold pack is that it can be used at any time on condition that ice cubes or iced water is available. The disadvantage of this cold pack is that the circulation of the fluid is for iced water. It is unable to be at a subzero temperature, causing a worse effect of cold compress therapy. Furthermore, it is unable to provide for depth cold compress therapy of muscle.

Accordingly, the inventor of the present invention has devoted himself based on his many years of practical experiences to the development of a new cold pack.

### SUMMARY OF THE INVENTION

The primary objective of the present invention is to provide a long-acting cold pack structure comprising at least a cold maintaining layer and a fluid layer. The cold maintaining layer is filled with gel-like cold maintaining material. The fluid layer is adapted for circulating low temperature fluid. With the cold maintaining layer, the cold pack keeps the cold maintaining layer at a subzero temperature after freezing in a refrigerator. With the circulation of the low temperature fluid in the fluid layer, the present invention permits the cold maintaining material in the cold maintaining layer to extend the time at a low temperature.

Another objective of the present invention is to provide a long-acting cold pack structure comprising a cold maintaining layer, a fluid layer and an air bag layer. The air bag layer is filled with air as desired to compress the cold maintaining layer for depth cold compress therapy.

With the cold maintaining layer, the fluid layer and the air bag layer, the cold pack of the present invention provides a better cold compress effect and extends the time of usage.

The gel-like cold maintaining material in the cold maintaining layer provides a soft and comfortable effect. Besides, the compound structure of the present invention provides a support effect to an affected part of a patient's body.

The cold maintaining layer is capable of absorbing. Accordingly, the low temperature of the fluid circulated within the fluid layer may be absorbed by the cold maintaining layer to extend the time of usage. Even though the circulation of the fluid is interrupted, the cold maintaining layer will keep the fluid at a low temperature for cold compress therapy.

### BRIEF DESCRIPTION OF THE DRAWINGS

Fig. 1 is a perspective view of the present invention;
Fig. 2 is a partially sectional view of the present invention;
Fig. 3 is a schematic view of the present invention in use; and
Fig. 4 is a cross-sectional view of the present invention in use.

### DETAILED DESCRIPTION OF THE PREFERRED EMBODIMENTS

Embodiments of the present invention will now be described, by way of example only, with reference to the accompanying drawings.

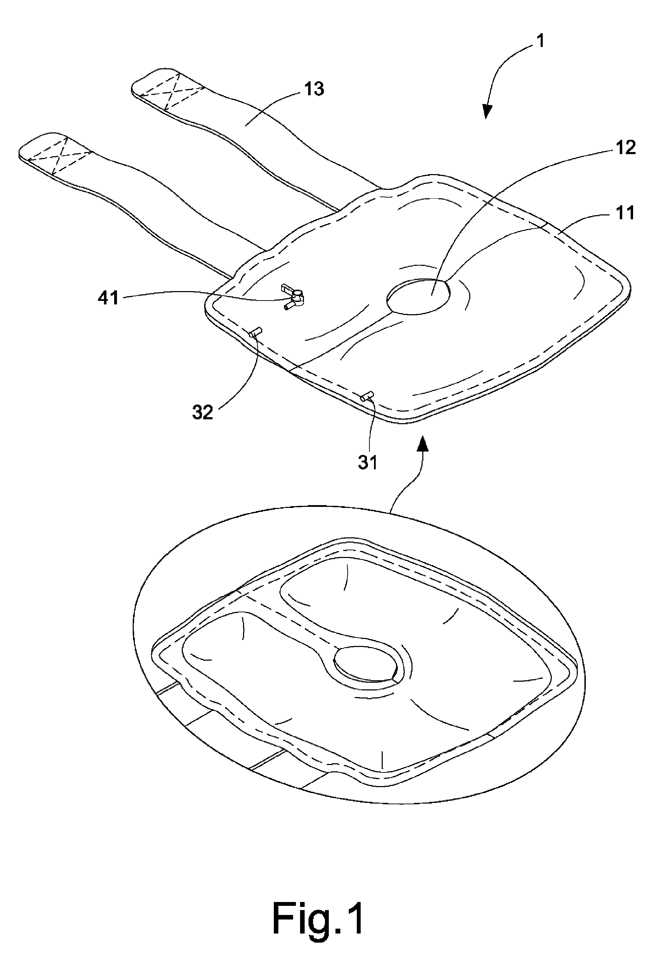

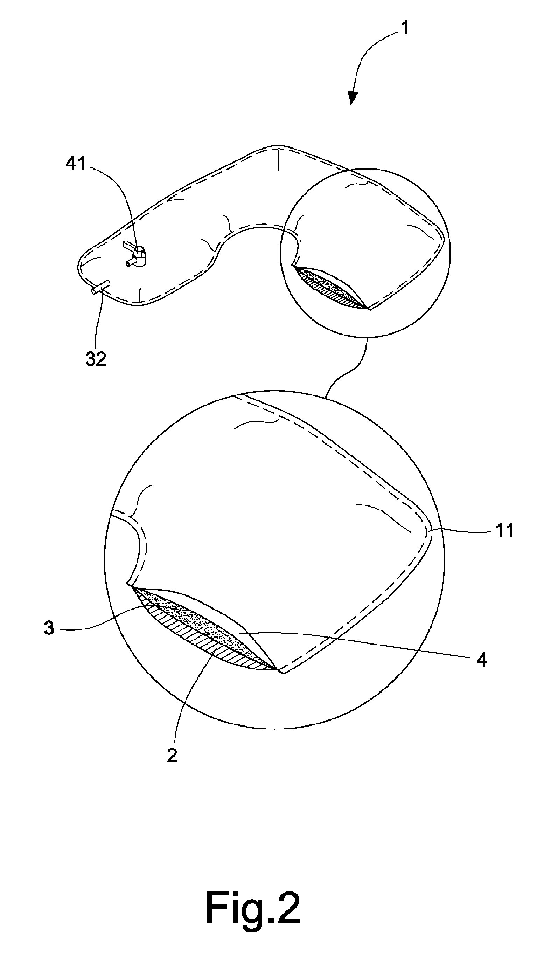

As shown in Figs. 1 through 3, a cold pack 1 according to a preferred embodiment of the present invention comprises a cold maintaining layer 2, a fluid layer 3, and an air bag layer 4.

The cold maintaining layer 2 is filled with gel-like cold maintaining material which is pliable.

The fluid layer 3 is attached to the cold maintaining layer 2, and has an inner space for pouring low temperature fluid therein. The fluid layer 3 is formed with an inlet 31 and an outlet 32. Each of the inlet 31 and the outlet 32 is provided with a water pipe 33 connected to a reservoir 34. The water pipe 33 is provided with a water pump for circulating the fluid. The reservoir 34 is filled with iced water.

The air bag layer 4 is attached to the fluid layer 3. The fluid layer 3 is sandwiched between the cold maintaining layer 2 and the air bag layer 4. The air bag layer 4 has an inner space for pouring air therein. The air bag layer 4 is provided with an air valve 41 which is connected with an air pipe 42 and an air pump 43. The air pump 43 is used to pour air into the air bag layer 4 through the air pipe 42. The air valve 41 is capable of opening and closing.

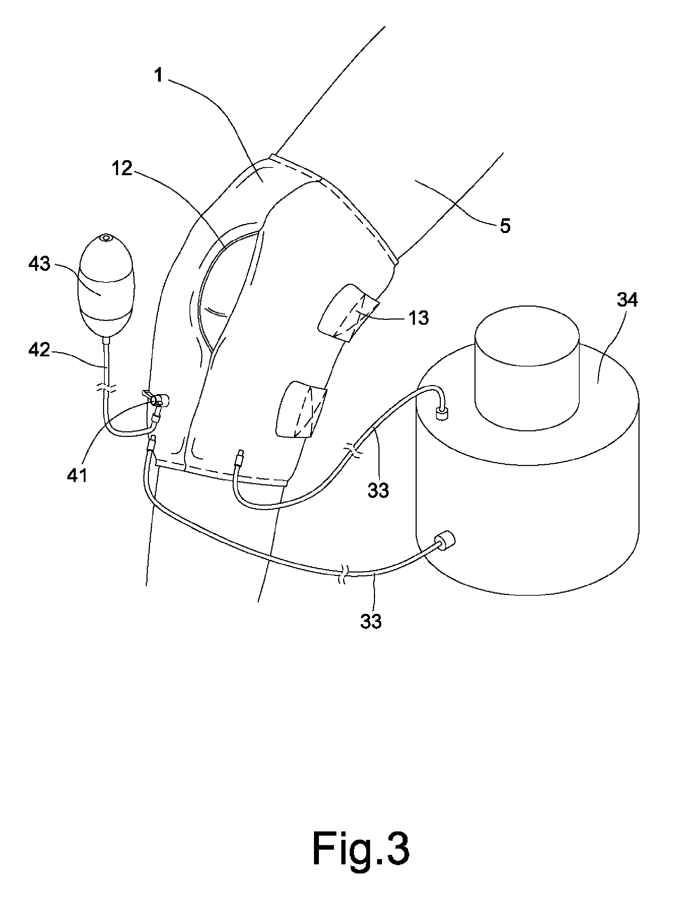

The cold pack 1 is composed of the cold maintaining layer 2, the fluid layer 3 and the air bag layer 4 which are stacked in sequence, and has a sealed portion 11 around the circumference of the cold pack 1. The cold pack 1 may be formed with a breach 12 and coupled with a connecting strap 13 for applying on a human body, as shown in Fig. 3. The shape of the present invention may be changed to fit any part of the human body.

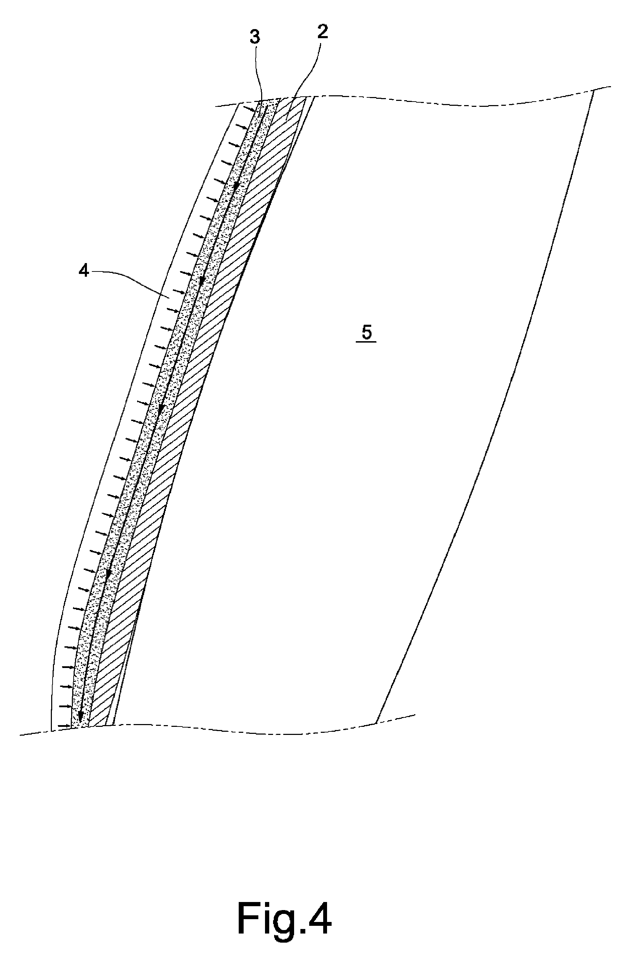

Before using the present invention, the cold pack 1 is freezing in a refrigerator to keep the cold maintaining layer 2 at a subzero temperature. To use the present invention, the cold pack 1 is taken out from the refrigerator for the following types of usage:
1. using the cold maintaining layer 2 for cold compress therapy;
2. not only using the cold maintaining layer 2 for cold compress therapy but also pouring iced fluid into the fluid layer 3 to circulate the fluid in the fluid layer 3 constantly for permitting the cold maintaining layer 2 to extend the time at a low temperature;
3. using the cold maintaining layer 2 and the fluid layer 3 for long-acting cold compress therapy and filling air into the air bag layer 4, as shown in Fig. 4, with the air bag layer 4 to compress the cold maintaining layer 2 tightly attached to the muscle 5 for depth cold compress therapy and the fluid layer 3 to supply the iced water constantly for permitting the cold maintaining layer 2 to extend the time at a low temperature.

Because of the present invention having the cold maintaining layer 2 and the fluid layer 3, if the cold pack 1 isn't frozen in the refrigerator in advance, the fluid layer 3 is adapted for pouring iced water to be circulated for urgent usage. That is to say, the present invention may use the cold maintaining layer 2 and the fluid layer 3 separately or both.

The surface of the cold pack of the present invention is made of cotton cloth having a waterproof inner layer, preventing congelation of sweat and providing better skin contact. The air bag layer 4 may be provided with an outer cotton layer to isolate hot and maintain cold, ensuring the low temperature not affected by the room temperature.

The gel-like cold maintaining material in the cold maintaining layer 2 is soft and comfortable for usage.

Although particular embodiments of the present invention have been described in detail for purposes of illustration, various modifications and enhancements may be made without departing from the spirit and scope of the present invention. Accordingly, the present invention is not to be limited except as by the appended claims.

## Claims

1. A long-acting cold pack (1) structure, comprising:
a cold maintaining layer (2) filled with cold maintaining material;
a fluid layer (3) attached to the cold maintaining layer (2), the fluid layer (3) having an inner space for pouring low temperature fluid, the fluid layer (3) further comprising at least an inlet (31); and
an air bag layer (4) attached to the fluid layer (3), the fluid layer (3) being sandwiched between the cold maintaining layer (2) and the air bag layer (4), the air bag layer (4) having an inner space for pouring air therein, the air bag layer (4) further comprising at least an air valve (41).

2. The long-acting cold pack (1) structure as claimed in claim 1, wherein the fluid layer (3) is provided with the inlet (31) and an outlet (32).

3. The long-acting cold pack (1) structure as claimed in claim 2, wherein each of the inlet (31) and the outlet (32) is provided with a water pipe (33) connected to a reservoir (34), the water pipe (33) being provided with a water pump for circulating the fluid.

4. The long-acting cold pack (1) structure as claimed in claim 1, wherein the air valve (41) is connected with an air pipe (42) and an air pump (43).

5. The long-acting cold pack (1) structure as claimed in claim 1, wherein the air valve (41) is capable of opening and closing.

6. The long-acting cold pack (1) structure as claimed in claim 1, further comprising a sealed portion (11) around the circumference of the cold pack (1).

7. The long-acting cold pack (1) structure as claimed in claim 1, further being formed with a break.

8. The long-acting cold pack (1) structure as claimed in claim 1, further comprising a connecting strap (13).

9. The long-acting cold pack (1) structure as claimed in claim 1, further having an outer surface made of cotton cloth with a waterproof inner layer.

10. The long-acting cold pack (1) structure as claimed in claim 4, wherein the air valve (41) is capable of opening and closing.
